# EUROPEAN PATENT APPLICATION

(11) **EP 4 487 952 A1**
(43) Date of publication of application: **08.01.2025**
(21) Application number: 22929299.0
(22) Date of filing: 02.03.2022
(51) Int. Cl.: B01J 31/06, C07C 45/30, C07C 201/12, C07C 47/54, C07C 49/78, C07C 47/02, C07C 49/403, C07C 205/44, C07B 41/06

(54) **CATALYST LOADED WITH TEMPO COMPOUND, AND PREPARATION METHOD THEREFOR AND USE THEREOF**

(71) Applicant: Asymchem Life Science (Tianjin) Co., Ltd, Tianjin 300457 (CN)
(72) Inventor: HONG, Hao, Morrisville North Carolina 27560 (US); JAMES, Gage, Morrisville North Carolina 27560 (US); XIAO, Yi, Tianjin 300457 (CN); PAN, Long, Tianjin 300457 (CN); SUN, Yuchen, Tianjin 300457 (CN); MA, Liteng, Tianjin 300457 (CN); ZHANG, Hangfei, Tianjin 300457 (CN); HOU, Bingyang, Tianjin 300457 (CN)
(74) Representative: Finetti, Claudia
(86) International application number: PCT/CN2022/078892
(87) International publication number: WO 2023/164848

(57) **Abstract**

Provided are a catalyst loaded with a TEMPO-type compound, and a preparation method and use thereof. The catalyst loaded with the TEMPO-type compound uses a polymer resin as a carrier, and the polymer resin is a polystyrene resin containing a chloromethyl or bromomethyl, herein the content of the chloromethyl or bromomethyl in the polymer resin is 0.5-5.0 mmol g⁻¹. The above polymer resin has a polystyrene framework and has a very low swelling degree in an organic solvent. In actual continuous production, the low swelling degree is beneficial for maintaining the rigidity of the resin, and the phenomena of softening, mutual extrusion, increased pressure (pressure holding) of a reaction apparatus and the like caused by resin expansion are prevented, such that it is ensured that the resin does not break; and the above resin has good tolerance to an oxidizing agent (such as sodium hypochlorite), and does not break and degrade after coming in contact with the oxidizing agent, and thus the cycle life of the loaded catalyst is ensured.

## Description

### Technical Field

The present disclosure relates to the field of catalysts, in particular to a catalyst loaded with a TEMPO compound, and a preparation method and use thereof.

### Background

In the pharmaceutical industry and chemical synthesis, oxidation reactions are a type of reactions with important significance. When an alcohol hydroxyl is oxidized, oxidizing agents involved may be divided into metallic oxidizing agents and non-metallic oxidizing agents according to whether it contains transition metal elements. The representative of the metallic oxidizing agents is a hexavalent chromium reagent, such as pyridinium chlorochromate (PCC), and active manganese dioxide. The main advantages are that the reagents are cheap and easy to obtain, and they have good selectivity. It may quantitatively oxidize primary alcohols into corresponding aldehydes without generating carboxylic acids. However, this type of the oxidizing agents may generate highly polluting heavy metal wastes during use, and its harmless treatment may lead to increased production costs. In addition, hexavalent chromium is a strong carcinogen, so its applications in the pharmaceutical industry are greatly limited. The non-metallic oxidizing agents typically include high-valent iodine reagents (such as 2-iodoxybenzoic acid (IBX) and Dess-Martin oxidizing agent), dimethyl sulfoxide reagents (usually used in combination with reagents such as dicyclohexylcarbodiimide, oxalyl chloride, and sulfur trioxide), and nitrogen oxide radical reagents (such as 2,2,6,6-tetramethylpiperidine oxide (TEMPO) or N-methylmorpholine oxide (NMO), usually used in combination with oxidizing agents such as chlorosuccinimide (NCS) or sodium hypochlorite). Compared to the metallic oxidizing agents, the non-metallic oxidizing agents generate less heavy metal wastes, while the selectivity is comparable. However, the high-valent iodine reagents are usually fed in equimolar or higher equivalents, its cost is expensive, and the improper storage of the reagent itself may lead to fires and explosions, thus the production cost is increased and safety hazards are introduced; the dimethyl sulfoxide reagents are reduced into dimethyl sulfide during use, it has foul odor and toxicity, and when used in combination with the oxalyl chloride, carbon monoxide is also generated; and in contrast, when the nitrogen oxide radical reagents are used, especially TEMPO compounds, sodium hypochlorite, NCS, and even the air may be used as oxidizing agents. The by-products have relatively low toxicity, are more convenient to treat, and cause less environmental burden. Thus, it is a type of green chemical reactions and has significant advantages in the chemical production.

At present, most of the reported cases of catalytic oxidation of the TEMPO compounds in documents are batch reactions, its process is that the TEMPO compounds are dissolved in reaction solvents and participate in homogeneous catalytic reactions. In the actual production, this batch reaction is often accompanied with the occurrence of side reactions (such as excessive oxidation of the primary alcohols), and many problems are often encountered when the reaction scale is enlarged; in addition, the price of the TEMPO compounds themselves is still relatively high, so for cost considerations, it is still necessary to design a recovery step for the TEMPO compounds after the reactions, to prevent its loss and prevent product contamination simultaneously. Since the oxidation reaction of alcohols is an exothermic reaction, it is even more important to consider the heat dissipation problems of reactors in the batch reactions, otherwise the accumulation of heat may become a safety hazard.

Compared to the batch reactions, continuous reactions are strong in controllability and good in safety. During the continuous reactions, the TEMPO compounds may be immobilized on polymer carriers (such as a resin) to catalyze the reactions, it is convenient for recovery and also prevents contamination of products. A plurality of embodiments of TEMPO compound immobilized resins are already reported in previous documents, but in practical use, the resins often gradually swell and break in organic solvents and oxidizing environments, leading to reactor blockage and increased back pressure. The direct result is a significant decrease in substrate conversion rate during catalyst cycling applications, and ultimately leading to insufficient cycling times, thus its practical applications are limited to a certain extent.

### Summary

A main purpose of the present disclosure is to provide a catalyst loaded with a TEMPO compound, and a preparation method and use thereof, as to solve problems of swelling and breakage of TEMPO compound immobilized resins during actual use in existing technologies.

In order to achieve the above purpose, according to one aspect of the present disclosure, a catalyst loaded with TEMPO-type compounds for oxidation reactions is provided, the catalyst uses a polymer resin as a carrier, and the polymer carrier is a polystyrene resin containing a chloromethyl or bromomethyl, herein the content of the chloromethyl or bromomethyl is 0.5-5.0 mmol g⁻¹.

Further, the above polymer resin is selected from one or more of a group consisting of resins with trademarks LX-B14, LX-207, LX-SS02, and LX-1000ME, and a Merrifield chloromethylated resin.

Further, the TEMPO-type compound includes one or more of hydroxyl-substituted TEMPO, amino-substituted TEMPO, and carbonyl-substituted TEMPO; and preferably, the TEMPO compound is the hydroxyl-substituted TEMPO, and more preferably, the TEMPO-type compound is 4-hydroxy-2,2,6,6-tetramethylpiperidine-1-oxide.

Further, the TEMPO-type compound is loaded on the polymer resin in the form of a covalent bond.

In order to achieve the above purpose, according to one aspect of the present disclosure, a preparation method for the above catalyst is provided, and the preparation method includes: in an organic solvent, a phase transfer catalyst is used to catalyze a substitution reaction of a polymer resin and a TEMPO-type compound, to obtain the catalyst.

Further, the usage amount of the TEMPO-type compound relative to the polymer resin is 4 mmol-12 mmol per gram of dry weight polymer resin; and preferably the temperature of the substitution reaction is 50-90°C, and the time of the substitution reaction is 2-3 days.

Further, the phase transfer catalyst is a tetrabutyl quaternary ammonium compound, preferably the tetrabutyl quaternary ammonium compound is selected from one or more of tetrabutyl ammonium hydroxide, tetrabutyl ammonium fluoride, tetrabutyl ammonium chloride, and tetrabutyl ammonium bromide; preferably inorganic alkali aqueous solution is added in the reaction, preferably the inorganic alkali aqueous solution is selected from one or more of sodium hydroxide solution and potassium hydroxide solution, and preferably the molar ratio of the tetrabutyl quaternary ammonium compound and the inorganic alkali is 1 :50-1 :400; and preferably the concentration of the inorganic alkali aqueous solution is 2-4 mol L⁻¹.

Further, the organic solvent is selected from one or more of toluene, chlorobenzene, and nitrobenzene.

Further, the preparation method further includes: washing and drying the catalyst, preferably ethanol is used as washing solution for the washing.

Further, a blast oven or an infrared lamp is used for the drying, the drying temperature is 60-100°C, and the drying time is 6-24 h.

According to another aspect of the present disclosure, use of the above catalyst loaded with the TEMPO-type compound is provided, and the use includes using the catalyst to catalyze an oxidation reaction, preferably the oxidation reaction is an oxidation reaction using alcohol as a substrate.

Further, the use includes using the catalyst to catalyze the alcohol for a continuous oxidation reaction, and preferably the use includes: continuously feeding an alcohol-containing substrate raw material and oxidizing solution into a continuous reactor to oxidize alcohol in the alcohol-containing substrate raw material, herein the continuous reactor is filled with the catalyst, the oxidizing solution is phosphate buffer solution (PBS) with sodium hypochlorite, the concentration of the sodium hypochlorite in the oxidizing solution is 0.35-0.80 mol/L, the concentration of the alcohol in the alcohol-containing substrate raw material is 0.10-0.40 mol/L, preferably the flow rate of the alcohol-containing substrate raw material is 0.13-1.00 g/min, and preferably the flow rate of the oxidizing solution is 0.15-1.20 g/min.

Further, the above alcohol is primary alcohol or secondary alcohol, preferably benzyl alcohol, 1-phenylethanol, n-hexanol, cyclohexanol, or p-nitrobenzyl alcohol.

Further, the concentration of the sodium hypochlorite in the oxidizing solution is 0.50-0.60 mol/L, the concentration of the alcohol in the alcohol-containing substrate raw material is 0.10-0.30 mol/L, preferably the flow rate of the alcohol-containing substrate raw material is 0.20-0.52 g/min, and preferably the flow rate of the oxidizing solution is 0.30-0.60 g/min.

Further, the retention time of the alcohol-containing substrate raw material in the continuous reactor is 30-60 min.

By applying technical schemes of the present disclosure, the catalyst loaded with the TEMPO-type compound in the present disclosure forms a heterogeneous catalyst by loading the TEMPO-type compound on the polymer carrier, it is easy to recover, prevents the product contamination, and may be reused without significant loss of activity. It may also be loaded into the continuous reactor and applied to the continuous chemical production, thereby a lot of material and labor costs are saved, heat transfer conditions are improved to increase safety, and the generation and discharge of wastes are reduced. In the present disclosure, the selected commercialized porous polymer resin carriers such as LX-B14, LX-207, LX-SS02, LX-1000ME, and Merrifield chloromethylated resin have a polystyrene framework, especially the above resin has a very low degree of swelling in the organic solvent. In the actual continuous production, the low swelling degree is beneficial for maintaining the rigidity of the resin, and the phenomena of softening, mutual extrusion, increased back pressure of the reaction apparatus and the like caused by resin expansion are prevented, such that it is ensured that the resin does not break. At the same time, the polystyrene framework of the above resin is relatively stable in the oxidizing environment and has good tolerance to an oxidizing agent (such as the sodium hypochlorite), and does not break and degrade after coming in contact with the oxidizing agent. In addition, these polymer resin carriers also have the advantages of high specific surface area, good strength, and low cost, thus it is suitable for large-scale industrial applications and has a long cycle life.

### Detailed Description of the Embodiments

It should be noted that, in the case without conflicting, embodiments in the present disclosure and features in the embodiments may be combined with each other. The present disclosure is described in detail below in combination with the embodiments.

As analyzed in the background of the present disclosure, the TEMPO-type compound immobilized resin in the existing technologies has the problems of easy swelling and breakage during the actual use. In order to solve these problems, the present disclosure provides a catalyst loaded with a TEMPO-type compound, and a preparation method and use thereof.

In a typical implementation mode of the present disclosure, a catalyst loaded with TEMPO-type compounds for oxidation reactions is provided, the catalyst uses a polymer resin as a carrier, and the polymer carrier is a polystyrene resin containing a chloromethyl or bromomethyl, herein the content of the chloromethyl or bromomethyl is 0.5-5.0 mmol g⁻¹; and preferably the content of the chloromethyl or bromomethyl is 0.6-4.1 mmol g⁻¹.

The catalyst loaded with the TEMPO-type compound in the present disclosure forms a heterogeneous catalyst by loading the TEMPO-type compound on the polymer carrier, it is easy to recover, prevents the product contamination, and may be reused without significant loss of activity. It may also be loaded into the continuous reactor and applied to the continuous chemical production, thereby a lot of material and labor costs are saved, heat transfer conditions are improved to increase safety, and the generation and discharge of wastes are reduced. The polymer resin in the present disclosure has a polystyrene framework and the above content of chloromethyl or bromomethyl, so the degree of swelling in the organic solvent is very low. In the actual continuous production, the low swelling degree is beneficial for maintaining the rigidity of the resin, and the phenomena of softening, mutual extrusion, increased pressure (pressure holding) of the reaction apparatus and the like caused by resin expansion are prevented, such that it is ensured that the resin does not break. At the same time, the polystyrene framework of the above polymer resin is relatively stable in the oxidizing environment and has good tolerance to an oxidizing agent (such as sodium hypochlorite), and does not break and degrade after coming in contact with the oxidizing agent. In addition, the above polymer resin carriers also have the advantages of high specific surface area, good strength, and low cost, thus it is suitable for large-scale industrial applications and has a long cycle life.

In some embodiments, preferably the above polymer resin carrier is selected from one or more of LX-B14 of Xi'an Sunresin Science and Technology, LX-207 of Xi'an Sunresin Science and Technology, LX-SS02 of Xi'an Sunresin Science and Technology, LX-1000ME, and Merrifield chloromethylated resin, and more preferably, the above polymer resin carrier is LX-B14 of Xi'an Sunresin Science and Technology. These functionalized resins have high reactivity, are easy to modify, and are cheap in price. Herein, the bromomethyl content in LX-B14 of Xi'an Sunresin Science and Technology is 3.5 mmol g⁻¹, the bromomethyl content in LX-207 of Xi'an Sunresin Science and Technology is 4.1 mmol g⁻¹, the chloromethyl content in LX-SS02 of Xi'an Sunresin Science and Technology is 1.2 mmol g⁻¹, the chloromethyl content in LX-1000ME is 0.6 mmol g⁻¹, and the chloromethyl content in the Merrifield chloromethylated resin is 1.4 mmol g⁻¹. After experimental exploration by the inventor, LX-B14 has the relatively high reusability and catalytic activity.

In some embodiments, the present disclosure selects a TEMPO-type compound that is readily available on the market, and the above TEMPO-type compound includes one or more of hydroxyl-substituted TEMPO, amino-substituted TEMPO, and carbonyl-substituted TEMPO; and in order to save costs and facilitate modifications, preferably the TEMPO-type compound is the hydroxyl-substituted TEMPO.

The above polymer resins are all polymer resins with benzyl halogenated groups, so the high activity of the benzyl halogenated groups may be utilized to react with the TEMPO-type compound, to form a covalent bond. In some embodiments, the TEMPO-type compound is loaded on the polymer resin in the form of the covalent bond, and the mode of covalent bonding makes the TEMPO-type compound more firmly bound to the polymer resin.

In another typical implementation mode of the present disclosure, a preparation method for the above catalyst is provided, and the preparation method includes: in an organic solvent, a phase transfer catalyst is used to catalyze a substitution reaction of a polymer resin and a TEMPO-type compound, to obtain the catalyst.

The preparation method adopted in the present disclosure is simple, the raw materials are convenient and readily available, and the cost is low. The TEMPO-type compound loaded catalyst prepared in the present disclosure covalently binds the TEMPO-type compound to the polymer carrier, to form the heterogeneous catalyst. It not only eliminates a catalyst recovery step and prevents the product contamination, but also may be loaded into a coil reactor and applied to the continuous chemical production, thereby a lot of material and labor costs are saved, heat transfer conditions are improved to increase safety, and the generation and discharge of wastes are reduced. In the present disclosure, the selected commercialized porous polymer resin carriers such as LX-B14, LX-207, LX-SS02, LX-1000ME, and Merrifield chloromethylated resin have a polystyrene framework, and have a very low degree of swelling in the organic solvent. In the actual continuous production, the low swelling degree is beneficial for maintaining the rigidity of the resin, and the phenomena of softening, mutual extrusion, increased back pressure of the reaction apparatus and the like caused by resin expansion are prevented, such that it is ensured that the resin does not break. At the same time, the polystyrene framework is relatively stable in the oxidizing environment and has good tolerance to an oxidizing agent (such as sodium hypochlorite), and does not break and degrade after coming in contact with the oxidizing agent. In addition, these polymer resin carriers also have the advantages of high specific surface area, good strength, and low cost, thus it is suitable for large-scale industrial applications.

In order to make the reaction perform completely, in some embodiments, the usage amount of the TEMPO-type compound relative to the polymer resin is 4 mmol-12 mmol per gram of dry weight polymer resin; and preferably the temperature of the substitution reaction is 50-90°C, and the time of the substitution reaction is 2-3 days.

In some embodiments, a phase transfer catalyst is added in the reaction process to promote the reaction, and the phase transfer catalyst includes various tetrabutyl ammonium compounds, preferably one or more of tetrabutyl ammonium hydroxide, tetrabutyl ammonium fluoride, tetrabutyl ammonium chloride, or tetrabutyl ammonium bromide. In the reaction process, inorganic alkali aqueous solution is added to construct an alkaline environment, and preferably the inorganic alkali aqueous solution is selected from one or more of sodium hydroxide solution or potassium hydroxide solution; and because the inorganic alkali may only be dissolved in solvents such as water or ethanol, and the use of ethanol as a solvent tend to result in side reactions, preferably the inorganic alkali aqueous solution is used. At this moment, the phase transfer catalyst reacts with the inorganic alkali, to generate the tetrabutyl ammonium hydroxide, and it has certain solubility in both organic solvents and water. Therefore, hydroxide ions may be transferred to an organic phase, as to promote the reaction, and halogen ions and hydrogen ions generated by the reaction may be transferred to an aqueous phase. At this moment, the TEMPO-type compound replaces a halogen in a halogenated alkyl of the polymer resin and covalently binds to the polymer resin. The hydroxide ions in the inorganic alkali aqueous solution may neutralize the halogen ions and hydrogen ions transferred to the aqueous phase.

In addition, preferably the molar ratio of the tetrabutyl ammonium compound and the inorganic alkali is 1:50-1:400, and preferably the molar ratio of the tetrabutyl ammonium compound and the inorganic alkali is 1:100; and preferably the concentration of the inorganic alkali aqueous solution is 2-4 mol L⁻¹, and preferably the concentration of the inorganic alkali aqueous solution is 3 mol L⁻¹, so that the two provide the sufficient hydroxide ions that may enter the organic phase, as to improve the reaction efficiency.

There are no special limitations on the types of the organic solvents. As long as it is not miscible with the water, it may dissolve the TEMPO-type compound, and its own boiling point and the boiling point of an azeotrope with the water are higher than the reaction temperature. The organic solvent is selected from one or more of toluene, chlorobenzene, and nitrobenzene. In order to save costs, preferably the organic solvent is the toluene, and preferably the concentration of the TEMPO-type compound in the organic solvent is 0.3mol L⁻¹.

In the above reaction process, in addition to forming a chemical bond between the TEMPO-type compound and the polymer resin, other products may also be generated. In order to remove impurities from the catalyst and facilitate subsequent use, the preparation method further includes: washing and drying the catalyst, preferably the ethanol is used as washing solution for the washing.

In order to avoid damage to the structure of the catalyst due to the excessive temperature in the drying process, preferably a blast oven or an infrared lamp is used for the above drying, the drying temperature is 60-100°C, and the drying time is 6-24 h.

In another typical implementation mode of the present disclosure, use of the above catalyst is provided, and the use includes using the catalyst to catalyze an oxidation reaction, preferably the oxidation reaction is an oxidation reaction using alcohol as a substrate.

The catalyst loaded with the TEMPO-type compound in the present disclosure is applied for the catalytic oxidation reaction. In the continuous chemical reaction, it may stably catalyze the conversion of the substrate alcohol into aldehydes and ketones, and the polymer resin carrier after the reaction may remain intact, unbroken or degraded after long-term applications. At the same time, by loading the TEMPO-type compound on the polymer resin, the mixing of the TEMPO-type compound and a product system is avoided, subsequent treatment operations are reduced, the cost is low, and the safety is improved.

Compared to batch reactions, the continuous reaction is stronger in controllability and better in safety. When the continuous reaction is performed, the catalyst loaded with the TEMPO-type compound may be used, and the catalyst is easy to recover and may also prevent the product contamination. In some embodiments, the above use of the present disclosure includes using the above catalyst loaded with the TEMPO-type compound to catalyze the alcohol for the continuous oxidation reaction, and preferably the above application includes: continuously feeding an alcohol-containing substrate raw material and oxidizing solution into a continuous reactor to oxidize alcohol in the alcohol-containing substrate raw material, herein the continuous reactor is filled with the catalyst, the oxidizing solution is phosphate buffer solution (PBS) added with sodium hypochlorite, the concentration of the sodium hypochlorite in the oxidizing solution is 0.35-0.80 mol/L, the concentration of the alcohol in the alcohol-containing substrate raw material is 0.10-0.40 mol/L, preferably the flow rate of the alcohol-containing substrate raw material is 0.13-1.00 g/min, and preferably the flow rate of the oxidizing solution is 0.15-1.20 g/min.

There are no special limitations on the alcohol in the present disclosure. In some embodiments, the alcohol is primary alcohol or secondary alcohol, preferably benzyl alcohol, 1-phenylethanol, n-hexanol, cyclohexanol, or p-nitrobenzyl alcohol.

The oxidizing solutions are that commonly used in this field. In some embodiments, the oxidizing solution is PBS with sodium hypochlorite. In the continuous chemical reaction, the substrate (the alcohol in the present disclosure) continuously enters the catalyst loaded with the TEMPO-type compound. Therefore, the concentration of sodium hypochlorite solution determines the amount of the oxidizing agent in contact with the substrate at a certain moment. Therefore, the concentration of the above alcohol, the concentration of the sodium hypochlorite, as well as the flow rate of the alcohol-containing substrate raw material and the flow rate of the oxidizing solution, are controlled to achieve the regulation of the oxidizing agent in contact with the substrate.

After research by the inventor, preferably the concentration of the sodium hypochlorite in the oxidizing solution is 0.50-0.60 mol/L. When the concentration is lower than this range, the conversion rate of the substrate is relatively low; and if the concentration is higher than this range and the oxidizing agent is excessive, it may easily cause side reactions and increase costs.

In some embodiments, the above alcohol-containing substrate raw material and the oxidizing solution are pumped into a continuous reactor by a reciprocating pump. For example, the continuous reactor includes one or more serially-connected cylindrical continuous reaction columns. More specifically, two serially-connected cylindrical continuous reaction columns, for example, may be arranged with a length of 25-100 cm and a diameter of 5-10 cm.

After research by the inventor, it is found that the concentration of the alcohol in the alcohol-containing substrate raw material is 0.10-0.30 mol/L, preferably the flow rate of the alcohol-containing substrate raw material is 0.20-0.52 g/min, and preferably the flow rate of the oxidizing solution is 0.30-0.60 g/min. If the concentration of the substrate solution is too high or the flow rate of the substrate solution/oxidizing solution is too fast, it may reduce the conversion rate; and if the flow rate is too slow, there is a possibility of triggering the side reactions, so a moderate flow rate is selected.

In some embodiments, the retention time of the alcohol-containing substrate raw material in the continuous reactor is 30-60 min.

The present disclosure is further described in detail below in combination with specific embodiments, and these embodiments may not be understood as limiting the scope of protection claimed by the present disclosure.

### Embodiment 1

A preparation method for a catalyst loaded with a TEMPO-type compound was as follows:

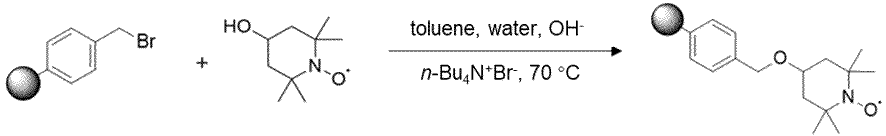

15 g of wet LX-B14 resin (the measured water content was 72%, and the actual dry weight was about 4.2 g), 6.21 g of 4-hydroxyl-2,2,6,6-tetramethylpiperidine-1-oxide (hydroxyl-substituted TEMPO), and 1.16 g of tetrabutyl ammonium bromide were taken, and added to a 500 mL four-necked flask. 120 mL of 3 mol L⁻¹ sodium hydroxide solution and 120 mL of toluene were added to the flask. A mechanical stirrer was installed and used to stir the mixture at 250 rpm. The reaction was heated at 70°C for 3 days. The resin obtained was washed with ethanol until there was no orange yellow color in washing solution. The resin was dried at 80 °C for 16 h, and then stored. The catalyst loaded with the TEMPO-type compound obtained was named as LX-B14-TEMPO.

### Embodiment 2

The difference from Embodiment 1 was that 11.5 g of wet LX-207 resin (the measured water content was 65%, and the actual dry weight was about 4.0 g) were used. The catalyst loaded with the TEMPO-type compound obtained was named as LX-207-TEMPO.

### Embodiment 3

The difference from Embodiment 1 was that 7 g of wet LX-SS02 resin (the measured water content was 41%, and the actual dry weight was about 4.0g) were used. The catalyst loaded with the TEMPO-type compound obtained was named as LX-SS02-TEMPO.

### Embodiment 4

The difference from Embodiment 1 was that 7.2 g of wet LX-1000ME resin (the measured water content was 45%, and the actual dry weight was about 4.1 g) were used. The catalyst loaded with the TEMPO-type compound obtained was named as LX-1000ME-TEMPO.

### Embodiment 5

The difference from Embodiment 1 was that 4 g of dry Merrifield chloromethylated resin (Supplier: Xi'an Sunresin Science and Technology New Material Co., Ltd.) were used. The catalyst loaded with the TEMPO-type compound obtained was named as Merrifield chloromethylated resin-TEMPO.

### Embodiment 6

15 g of wet LX-B14 resin (the measured water content was 72%, and the actual dry weight was about 4.2 g) and 6.21 g of 4-hydroxyl-2,2,6,6-tetramethylpiperidine-1-oxide (hydroxyl-substituted TEMPO) were taken, and added to a 500 mL four-necked flask. 120 mL of 3 mol L⁻¹ sodium hydroxide solution, 2.33 g of 40% aqueous solution of tetrabutyl ammonium hydroxide, and 120 mL of toluene were added to the flask. A mechanical stirrer was installed and used to stir the mixture at 250 rpm. The reaction was heated at 70°C for 3 days. The resin obtained was washed with ethanol until there was no orange yellow color in washing solution. The resin was dried at 80°C for 16 h, and then stored. The catalyst loaded with the TEMPO-type compound obtained was named as LX-B14-TEMPO-2.

### Embodiment 7

The difference from Embodiment 1 was that it was heated at 90°C for 3 days.

### Embodiment 8

The difference from Embodiment 1 was that it was heated at 50 °C for 3 days.

### Embodiment 9

The difference from Embodiment 1 was that 2.89 g of 4-hydroxyl-2,2,6,6-tetramethylpiperidine-1-oxide (hydroxyl-substituted TEMPO) was added.

### Embodiment 10

The difference from Embodiment 1 was that 8.67 g of 4-hydroxyl-2,2,6,6-tetramethylpiperidine-1-oxide (hydroxyl-substituted TEMPO) was added.

### Embodiment 11

The difference from Embodiment 1 was that 1.44 g of 4-hydroxyl-2,2,6,6-tetramethylpiperidine-1-oxide (hydroxyl-substituted TEMPO) was added.

### Embodiment 12

The difference from Embodiment 1 was that 11.55 g of 4-hydroxyl-2,2,6,6-tetramethylpiperidine-1-oxide (hydroxyl-substituted TEMPO) was added.

### Embodiment 13: LX-B14-TEMPO catalyzed oxidation reaction (batch reaction using benzyl alcohol as model substrate)

Firstly, buffered sodium hypochlorite stock solution was prepared. 40 mL of commercially available sodium hypochlorite solution (about 10 wt%) was diluted to 100 mL with 0.2 mol L⁻¹ phosphate buffer solution (PBS, pH=7.0); during the experiment, 2.5 mL of dichloromethane was added to a 15 mL centrifuge tube, and 100 µL of benzyl alcohol (model substrate) was added. The tube was shaken until the benzyl alcohol has dissolved to form a homogeneous solution. 3.75 mL of the above sodium hypochlorite stock solution was added, and then 0.1 mL of 1 mol L⁻¹ sodium bromide solution was added. The tube was shaken gently. Finally, 150 mg of the LX-B14-TEMPO loaded resin from Embodiment 1 was added. The centrifuge tube was closed tightly and it was placed in an ice bath and shaken continuously. After 1 h, an appropriate amount of the dichloromethane phase was taken, diluted with a solvent and sent for high performance liquid chromatography (HPLC) analysis. After reaction, the resin was filtrated, washed with dichloromethane and reused in the next cycle to evaluate its reusability. The conversion rate of each cycle was shown in Table 1:

**Table 1**

| Reaction times | 1 | 2 | 3 | 5 | 10 | 15 | 20 |
|---|---|---|---|---|---|---|---|
| Conversion rate of substrate | 100% | 100% | 100% | 98.39% | 96.80% | 96.40% | 91.22% |

### Embodiments 14 to 19

The catalyst loaded with the TEMPO-type compound prepared from Embodiments 7-12 was used to perform a catalytic oxidation reaction (batch reaction using benzyl alcohol as a model substrate).

Steps were the same as Embodiment 13, and the conversion rate of each reaction was shown in Table 2:

**Table 2**

| Reaction times | 1 | 2 | 3 | 5 | 10 | 15 | 20 |
|---|---|---|---|---|---|---|---|
| Embodiment 14 | 100% | 100% | 99.30% | 97.88% | 97.31% | 95.52% | 82.09% |
| Embodiment 15 | 100% | 97.27% | 95.47% | 92.83% | 85.16% | 81.89% | 73.03% |
| Embodiment 16 | 100% | 93.81% | 91.39% | 89.82% | 86.28% | 85.01% | 75.13% |
| Embodiment 17 | 100% | 100% | 100% | 99.07% | 97.04% | 97.65% | 86.31% |
| Embodiment 18 | 100% | 90.37% | 83.15% | 81.81% | 75.34% | 61.19% | 47.02% |
| Embodiment 19 | 100% | 100% | 99.86% | 98.81% | 96.29% | 96.57% | 87.73% |

### Embodiment 20: LX-B14-TEMPO catalyzed oxidation reaction (batch reaction using 1-phenylethanol as model substrate)

Firstly, buffered sodium hypochlorite stock solution was prepared. 40 mL of commercially available sodium hypochlorite solution (about 10 wt%) was diluted to 100 mL with 0.2 mol L⁻¹ PBS (pH=7.0); during the experiment, 2.5 mL of dichloromethane was added to a 15 mL centrifuge tube, and 120 µL of 1-phenylethanol (racemate, model substrate) was added. The tube was shaken until the 1-phenylethanol has dissolved to form a homogeneous solution. 3.75 mL of the above sodium hypochlorite stock solution was added, and then 0.1 mL of 1 mol L⁻¹ sodium bromide solution was added. The tube was shaken gently. Finally, 150 mg of the LX-B14-TEMPO loaded resin from Embodiment 1 was added. The centrifuge tube was closed tightly, and it was placed in an ice bath and shaken continuously. After 1 h, an appropriate amount of the dichloromethane phase was taken, diluted with a solvent and sent for HPLC analysis. After reaction, the resin was filtrated, washed with dichloromethane and reused in the next cycle to evaluate its reusability. The conversion rate of each cycle was shown in Table 3:

**Table 3**

| Reaction times | 1 | 2 | 3 | 5 | 10 | 15 | 20 |
|---|---|---|---|---|---|---|---|
| Conversion rate of substrate | 100% | 100% | 98.71% | 97.25% | 96.79% | 93.54% | 93.13% |

### Embodiment 21: LX-B14-TEMPO catalyzed oxidation reaction (batch reaction using n-hexanol as model substrate)

Firstly, buffered sodium hypochlorite stock solution was prepared. 40 mL of commercially available sodium hypochlorite solution (about 10wt%) was diluted to 100 mL with 0.2 mol L⁻¹ PBS (pH=7.0); during the experiment, 2.5 mL of dichloromethane was added to a 15 mL centrifuge tube, and 125 µL of n-hexanol (model substrate) was added. The tube was shaken until the n-hexanol has dissolved to form a homogeneous solution. 3.75 mL of the above sodium hypochlorite stock solution was added, and then 0.1 mL of 1 mol L⁻¹ sodium bromide solution was added. The tube was shaken gently. Finally, 150 mg of the LX-B14-TEMPO loaded resin from Embodiment 1 was added. The centrifuge tube was closed tightly, and it was placed in an ice bath and shaken continuously. After 1 h, an appropriate amount of the dichloromethane phase was taken, diluted with a solvent and sent for HPLC analysis. After reaction, the resin was filtrated, washed with dichloromethane and reused in the next cycle to evaluate its reusability. The conversion rate of each cycle was shown in Table 4:

**Table 4**

| Reaction times | 1 | 2 | 3 | 5 | 10 | 15 | 20 |
|---|---|---|---|---|---|---|---|
| Conversion rate of substrate | 100% | 100% | 97.23% | 96.91% | 96.42% | 94.47% | 94.09% |

### Embodiment 22: LX-B14-TEMPO catalyzed oxidation reaction (batch reaction using cyclohexanol as model substrate)

Firstly, buffered sodium hypochlorite stock solution was prepared. 40 mL of commercially available sodium hypochlorite solution (about 10wt%) was diluted to 100 mL with 0.2 mol L⁻¹ PBS (pH=7.0); during the experiment, 2.5 mL of dichloromethane was added to a 15 mL centrifuge tube, and 105 µL of cyclohexanol (model substrate) was added. The tube was shaken until the cyclohexanol has dissolved to form a homogeneous solution. 3.75 mL of the above sodium hypochlorite stock solution was added, and then 0.1 mL of 1 mol L⁻¹ sodium bromide solution was added. The tube was shaken gently. Finally, 150 mg of the LX-B14-TEMPO loaded resin from Embodiment 1 was added. The centrifuge tube was closed tightly, and it was placed in an ice bath and shaken continuously. After 1 h, an appropriate amount of the dichloromethane phase was taken, diluted with a solvent and sent for HPLC analysis. After reaction, the resin was filtrated, washed with dichloromethane and reused in the next cycle to evaluate its reusability. The conversion rate of each cycle was shown in Table 5:

**Table 5**

| Reaction times | 1 | 2 | 3 | 5 | 10 | 15 | 20 |
|---|---|---|---|---|---|---|---|
| Conversion rate of substrate | 95.32% | 94.87% | 93.46% | 92.69% | 91.88% | 91.15% | 91.09% |

### Embodiment 23: LX-B14-TEMPO catalyzed oxidation reaction (batch reaction using p-nitrobenzyl alcohol as model substrate)

Firstly, buffered sodium hypochlorite stock solution was prepared. 40 mL of commercially available sodium hypochlorite solution (about 10wt%) was diluted to 100 mL with 0.2 mol L⁻¹ PBS (pH=7.0); during the experiment, 2.5 mL of dichloromethane was added to a 15 mL centrifuge tube, and 150 mg of p-nitrobenzyl alcohol (model substrate) was added. The tube was shaken until the p-nitrobenzyl alcohol has dissolved to form a homogeneous solution. 3.75 mL of the above sodium hypochlorite stock solution was added, and then 0.1 mL of 1 mol L⁻¹ sodium bromide solution was added. The tube was shaken gently. Finally, 150 mg of the LX-B14-TEMPO loaded resin from Embodiment 1 was added. The centrifuge tube was closed tightly, and it was placed in an ice bath and shaken continuously. After 1 h, an appropriate amount of the dichloromethane phase was taken, diluted with a solvent and sent for HPLC analysis. After reaction, the resin was filtrated, washed with dichloromethane and reused in the next cycle to evaluate its reusability. The conversion rate of each cycle was shown in Table 6:

**Table 6**

| Reaction times | 1 | 2 | 3 | 5 | 10 | 15 | 20 |
|---|---|---|---|---|---|---|---|
| Conversion rate of substrate | 94.10% | 93.58% | 95.03% | 92.04% | 91.86% | 91.62% | 90.99% |

### Embodiment 24: LX-207-TEMPO catalyzed oxidation reaction (batch reaction using benzyl alcohol as model substrate)

Firstly, buffered sodium hypochlorite stock solution was prepared. 40 mL of commercially available sodium hypochlorite solution (about 10wt%) was diluted to 100 mL with 0.2 mol L⁻¹ PBS (pH=7.0); during the experiment, 2.5 mL of dichloromethane was added to a 15 mL centrifuge tube, and 100 µL of benzyl alcohol (model substrate) was added. The tube was shaken until the benzyl alcohol has dissolved to form a homogeneous solution. 3.75 mL of the above sodium hypochlorite stock solution was added, and then 0.1 mL of 1 mol L⁻¹ sodium bromide solution was added. The tube was shaken gently. Finally, 150 mg of the LX-207-TEMPO loaded resin from Embodiment 2 was added. The centrifuge tube was closed tightly, and it was placed in an ice bath and shaken continuously. After 1 h, an appropriate amount of the dichloromethane phase was taken, diluted with a solvent and sent for HPLC analysis. After reaction, the resin was filtrated, washed with dichloromethane and reused in the next cycle to evaluate its reusability. The conversion rate of each cycle was shown in Table 7:

**Table 7**

| Reaction times | 1 | 2 | 3 | 5 | 10 | 15 | 20 |
|---|---|---|---|---|---|---|---|
| Conversion rate of substrate | 100% | 96.47% | 96.16% | 95.42% | 92.83% | 88.65% | 79.12% |

### Embodiment 25: LX-SS02-TEMPO catalyzed oxidation reaction (batch reaction using benzyl alcohol as model substrate)

Firstly, buffered sodium hypochlorite stock solution was prepared. 40 mL of commercially available sodium hypochlorite solution (about 10wt%) was diluted to 100 mL with 0.2 mol L⁻¹ PBS (pH=7.0); during the experiment, 2.5 mL of dichloromethane was added to a 15 mL centrifuge tube, and 100 µL of benzyl alcohol (model substrate) was added. The tube was shaken until the benzyl alcohol has dissolved to form a homogeneous solution. 3.75 mL of the above sodium hypochlorite stock solution was added, and then 0.1 mL of 1 mol L⁻¹ sodium bromide solution was added. The tube was shaken gently. Finally, 150 mg of the LX-SS02-TEMPO loaded resin from Embodiment 3 was added. The centrifuge tube was closed tightly, and it was placed in an ice bath and shaken continuously. After 1 h, an appropriate amount of the dichloromethane phase was taken, diluted with a solvent and sent for HPLC analysis. After reaction, the resin was filtrated, washed with dichloromethane and reused in the next cycle to evaluate its reusability. The conversion rate of each cycle was shown in Table 8:

**Table 8**

| Reaction times | 1 | 2 | 3 | 5 | 10 | 15 | 20 |
|---|---|---|---|---|---|---|---|
| Conversion rate of substrate | 97.59% | 98.83% | 97.21% | 96.05% | 94.88% | 86.19% | 78.41% |

### Embodiment 26: LX-1000ME-TEMPO catalyzed oxidation reaction (batch reaction using benzyl alcohol as model substrate)

Firstly, buffered sodium hypochlorite stock solution was prepared. 40 mL of commercially available sodium hypochlorite solution (about 10wt%) was diluted to 100 mL with 0.2 mol L⁻¹ PBS (pH=7.0); during the experiment, 2.5 mL of dichloromethane was added to a 15 mL centrifuge tube, and 100 µL of benzyl alcohol (model substrate) was added. The tube was shaken until the benzyl alcohol has dissolved to form a homogeneous solution. 3.75 mL of the above sodium hypochlorite stock solution was added, and then 0.1 mL of 1 mol L⁻¹ sodium bromide solution was added. The tube was shaken gently. Finally, 150 mg of the LX-1000ME-TEMPO loaded resin from Embodiment 4 was added. The centrifuge tube was closed tightly, and it was placed in an ice bath and shaken continuously. After 1 h, an appropriate amount of the dichloromethane phase was taken, diluted with a solvent and sent for HPLC analysis. After reaction, the resin was filtrated, washed with dichloromethane and reused in the next cycle to evaluate its reusability. The conversion rate of each cycle was shown in Table 9:

**Table 9**

| Reaction times | 1 | 2 | 3 | 5 | 10 | 15 | 20 |
|---|---|---|---|---|---|---|---|
| Conversion rate of substrate | 100% | 97.84% | 98.94% | 96.64% | 91.76% | 85.43% | 83.39% |

### Embodiment 27: Merrifield chloromethylated resin-TEMPO catalyzed oxidation reaction (batch reaction using benzyl alcohol as model substrate)

Firstly, buffered sodium hypochlorite stock solution was prepared. 40 mL of commercially available sodium hypochlorite solution (about 10wt%) was diluted to 100 mL with 0.2 mol L⁻¹ PBS (pH=7.0); during the experiment, 2.5 mL of dichloromethane was added to a 15 mL centrifuge tube, and 100 µL of benzyl alcohol (model substrate) was added. The tube was shaken until the benzyl alcohol has dissolved to form a homogeneous solution. 3.75 mL of the above sodium hypochlorite stock solution was added, and then 0.1 mL of 1 mol L⁻¹ sodium bromide solution was added. The tube was shaken gently. Finally, 150 mg of the Merrifield chloromethylated resin-TEMPO loaded resin from Embodiment 5 was added. The centrifuge tube was closed tightly, and it was placed in an ice bath and shaken continuously. After 1 h, an appropriate amount of the dichloromethane phase was taken, diluted with a solvent and sent for HPLC analysis. After reaction, the resin was filtrated, washed with dichloromethane and reused in the next cycle to evaluate its reusability. The conversion rate of each cycle was shown in Table 10:

**Table 10**

| Reaction times | 1 | 2 | 3 | 5 | 10 | 15 | 20 |
|---|---|---|---|---|---|---|---|
| Conversion rate of substrate | 100% | 96.31% | 97.68% | 93.11% | 91.64% | 84.93% | 77.42% |

### Embodiment 28: LX-B14-TEMPO-2 catalyzed oxidation reaction (batch reaction using benzyl alcohol as model substrate)

Firstly, buffered sodium hypochlorite stock solution was prepared. 40 mL of commercially available sodium hypochlorite solution (about 10wt%) was diluted to 100 mL with 0.2 mol L⁻¹ PBS (pH=7.0); during the experiment, 2.5 mL of dichloromethane was added to a 15 mL centrifuge tube, and 100 µL of benzyl alcohol (model substrate) was added. The tube was shaken until the benzyl alcohol has dissolved to form a homogeneous solution. 3.75 mL of the above sodium hypochlorite stock solution was added, and then 0.1 mL of 1 mol L⁻¹ sodium bromide solution was added. The tube was shaken gently. Finally, 150 mg of the LX-B14-TEMPO loaded resin from Embodiment 1 was added. The centrifuge tube was closed tightly, and it was placed in an ice bath and shaken continuously. After 1 h, an appropriate amount of the dichloromethane phase was taken, diluted with a solvent and sent for HPLC analysis. After reaction, the resin was filtrated, washed with dichloromethane and reused in the next cycle to evaluate its reusability. The conversion rate of each cycle was shown in Table 11:

**Table 11**

| Reaction times | 1 | 2 | 3 | 5 | 10 | 15 | 20 |
|---|---|---|---|---|---|---|---|
| Conversion rate of substrate | 100% | 98.07% | 97.63% | 97.72% | 96.44% | 92.87% | 92.66% |

### Embodiment 29: LX-B14-TEMPO catalyzed oxidation reaction (continuous chemical reaction using benzyl alcohol as model substrate)

This continuous chemical reaction involved 2 types of raw material solution, herein solution A was solution of benzyl alcohol in dichloromethane, and its concentration was 0.2 mol L⁻¹; and solution B was buffered sodium hypochlorite solution. 40 mL of commercially available sodium hypochlorite solution (about 10wt%) was taken and diluted to 100 mL with 0.2 mol L⁻¹ PBS (pH=7.0). The concentration of the sodium hypochlorite was about 0.53 mol L⁻¹. After the LX-B14-TEMPO loaded resin from Embodiment 1 was wetted with dichloromethane, two cylindrical iron pipe columns with a diameter of 8 mm were filled with it. After being filled, both ends were blocked with a fine metal mesh. The length of each iron pipe column was 50 cm; and after being filled, the LX-B14-TEMPO resin catalyst in the iron pipe columns was blown dry with a nitrogen gas for later use.

During the experiment, two medium and low pressure reciprocating pumps were used to form a continuous chemical reaction system with the above iron pipe columns. A T-shaped joint in front of the column was used for mixing, the two columns were connected serially with a polytetrafluoroethylene pipe, and it was reacted at a room temperature.

The flow rate of the reciprocating pump might be: 0.26 g min⁻¹ for the solution A and 0.30 g min⁻¹ for the solution B.

After the flow rate was set, the reciprocating pump was started for feeding. At this flow rate, liquid was discharged from a discharge port after 90 min, and it was estimated from this that the retention time of the material in the column was about 40 min. After 4 h of the feeding, an effluent was taken for 10 min, and a dichloromethane phase was taken from the effluent, diluted and sent for HPLC analysis. After the reaction, the entire apparatus was rinsed with the dichloromethane and the residual dichloromethane in the iron pipe columns was blown clean with the nitrogen gas.

In this embodiment, the continuous reaction apparatus might continuously oxidize at least 1 mol of the benzyl alcohol to convert it into benzaldehyde, and the conversion rate of the substrate might be maintained at 90% or above. It was worth noting that the usage amount of LX-B14-TEMPO filled in this apparatus was only for small-scale testing. In the actual production, more resins might be loaded into a coil reactor for catalytic oxidation of the substrate in kilogram scale or above.

### Embodiment 30

The difference from Embodiment 29 was that the solution A was solution of benzyl alcohol in dichloromethane, and its concentration was 0.1 mol L⁻¹.

### Embodiment 31

The difference from Embodiment 29 was that the solution A was solution of benzyl alcohol in dichloromethane, and its concentration was 0.4 mol L⁻¹.

### Embodiment 32

The difference from Embodiment 29 was that a preparation method for the solution B was as follows: 20 mL of commercially available sodium hypochlorite solution (about 10wt%) was diluted to 100 mL with 0.2 mol L⁻¹ PBS (pH=7.0), and the concentration of the sodium hypochlorite was about 0.35 mol L⁻¹.

### Embodiment 33

The difference from Embodiment 29 was that a preparation method for the solution B was as follows: 60 mL of commercially available sodium hypochlorite solution (about 10wt%) was diluted to 100 mL with 0.2 mol L⁻¹ PBS (pH=7.0), and the concentration of the sodium hypochlorite was about 0.80 mol L⁻¹.

### Embodiment 34

The difference from Embodiment 29 was that the flow rate of the reciprocating pump was: 0.13 g min⁻¹ for the solution A and 0.15 g min⁻¹ for the solution B.

### Embodiment 35

The difference from Embodiment 29 was that the flow rate of the reciprocating pump was: 0.20 g min⁻¹ for the solution A and 0.30 g min⁻¹ for the solution B.

### Embodiment 36

The difference from Embodiment 29 was that the flow rate of the reciprocating pump was: 0.52 g min⁻¹ for the solution A and 0.60 g min⁻¹ for the solution B.

### Embodiment 37

The difference from Embodiment 29 was that the flow rate of the reciprocating pump was: 1.00 g min⁻¹ for the solution A and 1.20 g min⁻¹ for the solution B.

The substrate conversion rates of Embodiments 29 to 37 were shown in Table 12.

**Table 12**

| Embodiment | Substrate conversion rate |
|---|---|
| Embodiment 29 | 95% |
| Embodiment 30 | 89% |
| Embodiment 31 | 83% |
| Embodiment 32 | 79% |
| Embodiment 33 | 86% |
| Embodiment 34 | 84% |
| Embodiment 35 | 89% |
| Embodiment 36 | 83% |
| Embodiment 37 | 76% |

### Comparative example 1: 4-hydroxyl-2,2,6,6-tetramethylpiperidine-1-oxide (hydroxyl-substituted TEMPO) TEMPO-type compound catalyzed oxidation reaction (batch reaction using benzyl alcohol as model substrate)

Firstly, buffered sodium hypochlorite stock solution was prepared. 40 mL of commercially available sodium hypochlorite solution (about 10wt%) was diluted to 100 mL with 0.2 mol L⁻¹ PBS (pH=7.0); during the experiment, 2.5 mL of dichloromethane was added to a 15 mL centrifuge tube, and 100 µL of benzyl alcohol (model substrate) was added. The tube was shaken until the benzyl alcohol has dissolved to form a homogeneous solution. 3.75 mL of the above sodium hypochlorite stock solution was added, and then 0.1 mL of 1 mol L⁻¹ sodium bromide solution was added. The tube was shaken gently. Finally, 150 mg of 4-hydroxyl-2,2,6,6-tetramethylpiperidine-1-oxide (hydroxyl-substituted TEMPO-type compound) was added, the centrifuge tube was closed tightly, and it was placed in an ice bath and shaken continuously. After 1 h, an appropriate amount of the dichloromethane phase was taken, diluted with a solvent and sent for HPLC analysis. This conversion rate was calculated as 100%.

### Comparative example 2: Oxidation reaction catalyzed by chloromethyl styrene resin with low chloromethyl content (batch reaction using benzyl alcohol as model substrate)

### (1) Synthesis of resin with low chloromethyl content

A 250 mL four-necked flask was immersed in an ice bath, and 10.0 g (11.93 mL) of dimethoxymethane, 2.8 g (2.36 mL) of concentrated hydrochloric acid, and 3.8 g of trimeric formaldehyde were added sequentially. 3.5 g (1.87 mL) of concentrated sulfuric acid was dropwise added slowly to the flask. After being stirred uniformly, 2.6 g of a high crosslinked styrene resin (the crosslinking degree was about 75%) was added, it was stirred again for 1 h, and 0.8 g of anhydrous zinc chloride and 10 mL of sulfoxide chloride were added. The system was carefully refluxed overnight at a temperature slightly below 40 °C. After being refluxed overnight, the resin was filtered, washed with a large amount of ethanol and tetrahydrofuran (THF), and dried. The resin was named as DVB-st-Cl. After being represented, the chloromethyl content of the DVB-st-Cl resin was about 0.08 mmol/g.

### (2) TEMPO loading of resin

0.5 g of dry DVB-st-Cl resin balls, 0.62 g of 4-hydroxyl-2,2,6,6-tetramethylpiperidine-1-oxide (hydroxyl-substituted TEMPO), and 0.12 g of tetrabutyl ammonium bromide were taken, and added to a 100 mL four-necked flask. 15 mL of 3 mol L⁻¹ sodium hydroxide solution and 15 mL of toluene were added to the flask, a mechanical stirrer was installed, and it was stirred at 250 rpm, and heated at 70°C for 3 days. A resin obtained was washed with ethanol until there was no orange yellow color in washing solution, dried at 80°C for 16 h, and then stored. The catalyst loaded with the TEMPO-type compound obtained was named as DVB-st-CI-TEMPO.

### (3) Catalytic oxidation reaction

Firstly, buffered sodium hypochlorite stock solution was prepared. 40 mL of commercially available sodium hypochlorite solution (about 10wt%) was diluted to 100 mL with 0.2 mol L⁻¹ PBS (pH=7.0); during the experiment, 2.5 mL of dichloromethane was added to a 15 mL centrifuge tube, and 100 µL of benzyl alcohol (model substrate) was added. The tube was shaken until the benzyl alcohol has dissolved to form a homogeneous solution. 3.75 mL of the above sodium hypochlorite stock solution was added, and then 0.1 mL of 1 mol L⁻¹ sodium bromide solution was added. The tube was shaken gently. Finally, 150 mg of the above DVB-st-CI-TEMPO loaded resin was added, the centrifuge tube was closed tightly, and it was placed in an ice bath and shaken continuously. After 1 h, an appropriate amount of the dichloromethane phase was taken, diluted with a solvent and sent for HPLC analysis. After reaction, the resin was filtrated, washed with dichloromethane and reused in the next cycle to evaluate its reusability. The conversion rate of each cycle was shown in Table 13:

**Table 13**

| Reaction times | 1 | 2 | 3 | 5 | 10 | 15 | 20 |
|---|---|---|---|---|---|---|---|
| Conversion rate of substrate | 73.35% | 71.27% | 64.52% | 41.09% | 25.54% | 6.44% | 0.56% |

The content of the TEMPO-type compound in the catalyst loaded with the TEMPO-type compound is not easily represented directly. The inventor researches the catalytic kinetics of loaded catalysts for the LX-B14-TEMPO-type compound, and it is found from results that the catalytic effect per gram of the resin is equivalent to homogeneous solution of the TEMPO-type compound in 10⁻⁴ mmol/L orders of magnitude. Since the resin balls are insoluble in the solvent, it belongs to heterogeneous catalysts in the solution, and its catalytic behavior is significantly different from that of the homogeneous solution. Therefore, this data may not be directly equivalent to the content of the TEMPO-type compound on the resin.

From the above description, it may be seen that the embodiments of the present disclosure achieved the following technical effects: the present disclosure describes a heterogeneous catalyst by loading the TEMPO-type compound on the polymer carrier, which not only eliminates a catalyst recovery step and prevents the product contamination, but the heterogeneous catalyst may also be loaded into a coil reactor and applied to the continuous chemical production, thereby a lot of material and labor costs are saved, heat transfer conditions are improved to increase safety, and the generation and discharge of wastes are reduced. In the present disclosure, the selected commercialized porous polymer resin carriers such as LX-B14, LX-207, LX-SS02, LX-1000ME, and Merrifield chloromethylated resin have a polystyrene framework, especially the above resin has a very low degree of swelling in the organic solvent. In the actual continuous production, the low swelling degree is beneficial for maintaining the rigidity of the resin, and the phenomena of softening, mutual extrusion, increased back pressure of the reaction apparatus and the like caused by resin expansion are prevented, such that it is ensured that the resin does not break. At the same time, the polystyrene framework is relatively stable in the oxidizing environment and has good tolerance to an oxidizing agent (such as sodium hypochlorite), and does not break and degrade after coming in contact with the oxidizing agent. In addition, these polymer resin carriers also have the advantages of high specific surface area, good strength, and low cost, thus it is suitable for large-scale industrial applications and has a long cycle life.

The above are only preferred embodiments of the present disclosure and are not intended to limit the present disclosure. For those skilled in the art, the present disclosure may have various modifications and changes. Any modifications, equivalent replacements, improvements and the like made within the spirit and principles of the present disclosure shall be contained within the scope of protection of the present disclosure.

## Claims

1. A catalyst loaded with TEMPO-type compounds for oxidation reactions, wherein the catalyst uses a polymer resin as a carrier, the polymer resin is a polystyrene resin containing a chloromethyl or bromomethyl, herein the content of the chloromethyl or bromomethyl is 0.5-5.0 mmol g⁻¹.

2. The catalyst according to claim 1, wherein the polymer resin is selected from one or more of a group consisting of resins with trademarks LX-B14, LX-207, LX-SS02, and LX-1000ME, and a Merrifield chloromethylated resin.

3. The catalyst according to claim 1, wherein the TEMPO-type compound comprises one or more of a hydroxyl-substituted TEMPO, an amino-substituted TEMPO, and a carbonyl-substituted TEMPO; preferably, the TEMPO -types compound is 4-hydroxy-2,2,6,6-tetramethylpiperidine-1-oxide.

4. The catalyst according to claim 1, wherein the TEMPO-type compound is loaded on the polymer resin in the form of a covalent bond.

5. A preparation method for the catalyst according to any one of claims 1 to 4, wherein the preparation method comprises:
in an organic solvent, using a phase transfer catalyst to catalyze a substitution reaction of a polymer resin and a TEMPO-type compound, as to obtain the catalyst.

6. The preparation method according to claim 5, wherein the usage amount of the TEMPO-type compound relative to the polymer resin is 4 mmol to 12 mmol per gram of dry weight polymer resin; preferably the temperature of the substitution reaction is 50 to 90 °C, and the time of the substitution reaction is 2 to 3 days.

7. The preparation method according to claim 6, wherein the phase transfer catalyst is a tetrabutyl quaternary ammonium compound, preferably the tetrabutyl quaternary ammonium compound is selected from one or more of a tetrabutyl ammonium hydroxide, a tetrabutyl ammonium fluoride, a tetrabutyl ammonium chloride, and a tetrabutyl ammonium bromide; preferably inorganic alkali aqueous solution is added in the reaction, preferably the inorganic alkali aqueous solution is selected from one or more of sodium hydroxide solution and potassium hydroxide solution, and preferably the molar ratio of the tetrabutyl quaternary ammonium compound and the inorganic alkali is 1:50 to 1:400; and preferably the concentration of the inorganic alkali aqueous solution is 2 to 4 mol L⁻¹.

8. The preparation method according to any one of claims 5 to 7, wherein the organic solvent is selected from one or more of a toluene, a chlorobenzene, and a nitrobenzene.

9. The preparation method according to any one of claims 5 to 7, wherein the preparation method further comprises: washing and drying the catalyst, preferably ethanol is used as washing solution for the washing.

10. The preparation method according to claim 9, wherein a blast oven or an infrared lamp is used for the drying, preferably the drying temperature is 60 to 100 °C, and preferably the drying time is 6 to 24 h.

11. Use of the catalyst loaded with TEMPO-type compounds according to any one of claims 1 to 4, wherein the use comprises using the catalyst to catalyze an oxidation reaction, preferably the oxidation reaction is an oxidation reaction using alcohol as a substrate.

12. The use according to claim 11, wherein the use comprises using the catalyst to catalyze the alcohol for a continuous oxidation reaction, preferably the use comprises:
an alcohol-containing substrate raw material and oxidizing solution into a continuous reactor to oxidize alcohol in the alcohol-containing substrate raw material, herein the continuous reactor is filled with the catalyst, the oxidizing solution is phosphate buffer solution (PBS) with sodium hypochlorite, the concentration of the sodium hypochlorite in the oxidizing solution is 0.35-0.80 mol/L, the concentration of the alcohol in the alcohol-containing substrate raw material is 0.10-0.40 mol/L, preferably the flow rate of the alcohol-containing substrate raw material is 0.13-1.00 g/min, and preferably the flow rate of the oxidizing solution is 0.15-1.20 g/min.

13. The use according to claim 11 or 12, wherein the alcohol is primary alcohol or secondary alcohol, preferably benzyl alcohol, 1-phenylethanol, n-hexanol, cyclohexanol, or p-nitrobenzyl alcohol.

14. The use according to claim 12, wherein the concentration of the sodium hypochlorite in the oxidation liquid is 0.50 to 0.60 mol/L, and the concentration of the alcohol in the alcohol-containing substrate raw material is 0.10 to 0.30 mol/L, preferably the flow rate of the alcohol-containing substrate raw material is 0.20 to 0.52 g/min, and preferably the flow rate of the oxidizing solution is 0.30 to 0.60 g/min.

15. The use according to claim 12, wherein the retention time of the alcohol-containing substrate raw material in the continuous reactor is 30 to 60 min.
